(19) Europäisches Patentamt
European Patent Office
Office européen des brevets

(11) EP 1 329 501 A1

(12) EUROPEAN PATENT APPLICATION
published in accordance with Art. 158(3) EPC

(43) Date of publication:
**23.07.2003 Bulletin 2003/30**

(21) Application number: **01956958.1**

(22) Date of filing: **20.08.2001**

(51) Int Cl.[7]: **C12N 9/16**, C12N 15/55, C12N 1/21, C12N 1/19, C12N 5/10, C07K 16/40, C12Q 1/02, C12Q 1/68, A61K 31/711, A61K 38/46, A61K 39/395, A61P 43/00

(86) International application number:
**PCT/JP01/07106**

(87) International publication number:
**WO 02/031131 (18.04.2002 Gazette 2002/16)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **11.10.2000 JP 2000311015**

(71) Applicant: **MOCHIDA PHARMACEUTICAL CO., LTD.**
**Shinjuku-ku Tokyo 160-8515 (JP)**

(72) Inventors:
- **ARAI, Hiroyuki**
  **Tokyo 112-0002 (JP)**
- **AOKI, Junken**
  **Tokyo 154-0003 (JP)**

(74) Representative: **Woods, Geoffrey Corlett**
**J.A. KEMP & CO.,**
**Gray's Inn,**
**14 South Square**
**London WC1R 5JJ (GB)**

(54) **NOVEL PLA1**

(57) A novel phospholipase $A_1$ ($PLA_1$) having a substrate specificity for phosphatidic acid (PA); a peptide or a polypeptide originating in the novel $PLA_1$; a polynucleotide encoding the peptide or the polypeptide originating in the novel $PLA_1$; a process for producing the peptide or the polypeptide originating in the novel $PLA_1$; an antibody against the peptide or the polypeptide originating in the novel $PLA_1$; a method of identifying an inhibitor, an antagonist or a potentiator for the novel $PLA_1$ by using the same; a compound identified by this method; and medicinal compositions and a diagnostic method with the use of the same.

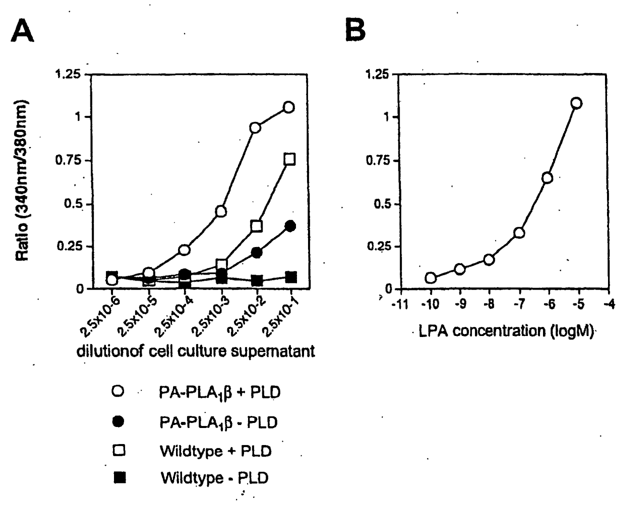


Fig.4

EP 1 329 501 A1

## Description

### Technical Field

This invention relates to a novel phospholipase $A_1$, which will be designated as "$PLA_1$" as well in the present specification. This invention more particularly relates to a peptide or polypeptide having the whole or a part of the novel $PLA_1$, to a recombinant vector having the polynucleotide, to a transformant transformed with the vector, to a method for the production of the peptide or polypeptide by using the transformant, to an antibody against the peptide or polypeptide, a method for identification of a compound by utilizing these materials, to the compound thus identified, to an inhibitor or potentiator interacting with the polypeptide or polynucleotide, to a pharmaceutical composition, to a method for the production thereof, to a method for the treatment using thereof, and to a method for the diagnosis of diseases relating to the phospholipase $A_1$.

### Background Art

$PLA_1$ is an enzyme that hydrolyses the first ester bonding in glycerol of glycerophospholipid. Up to now, an activity of this enzyme has been observed in various organs, and several types of the enzyme classified according to their substrate specificity have been reported. $PLA_1$s whose cDNA has been cloned include bee-poison $PLA_1$(Dolm1), PS-$PLA_1$ that specifically hydrolyses the first ester bonding in glycerol of phosphatidylserine (PS) and lysophosphatidylserine (lysoPS) (Japanese Patent Application Laid Open Hei.10(1998)-201479; Protein, Nucleic Acid and Enzyme, 44 1038-1042, 1999), and PA-$PLA_1$ in human testis that specifically hydrolyses the first ester bonding in glycerol of phosphatidic acid (J. Biol. Chem., 273, 5468-5477, 1998). Furthermore, molecules belonging to a lipase family are known to have $PLA_1$ activity in addition to an activity of decomposing triacylglycerol (FEBS Letters, 320, 145-149, 1993; Biochemistry, 32, 4702-4707, 1993; J. Biol. Chem., 272, 2192-2198, 1997). Although all of the $PLA_1$s belonging to the lipase family has been found to have a short Lid (B.B.A., 1376, 417-432, 1998; Biochemistry, 32, 4702-4707, 1993), their physiological significance has not yet been revealed. It is suggested that a sugar chain in the lipase molecule may possibly contribute to its activity.

One of the activities of $PLA_1$ is to decompose phospholipid. And one of the products originated from the decomposition, lysophosphatidic acid (LPA) (B.B.A., 1198, 185-196, 1994), is known to have many physiological activities and attention is focused on its biological utility (Cell Technology, 17 (5), 739-745, 1998). Main activities of LPA include blood pressure-raising activity (Lipids, 13, 572-574, 1978; platelet-aggregation activity (Am., J. Pathol., 96, 423-438, 1979), cell propagation-promoting activity (Cell, 59, 45-54, 1989). In addition to them, various activities of PLA have been reported such as those of promoting infiltration of cancer cells, cell adhesion, formation of stress fiber, induction of chemotaxis, involution of neurite, inhibition of apoptosis, and curing of wound (B.B.A., 1998, 185-196, 1994).

Human testis PA-$PLA_1$ is known to have a specificity for PA, and its cDNA has been cloned. This $PLA_1$ is an intracellular enzyme and is considered a determining factor of metabolic turnover of a fatty acid at the sn-1 position of PA located in the center of metabolism of phospholipid (J. Biol. Chem., 273, 5468-5477, 1998). It is also reported that human testis PA-$PLA_1$ will hydrolyze also phosphatidylethanolamine (PE) and phosphatidylinositol (PI) depending on reaction conditions.

One of the purposes of the present invention is to find a novel $PLA_1$ that catalyzes and produces LPA capable of being a causative agent of disadvantageous functions in certain aspects, and to enable the control of LPA in a living body.

### Disclosure of Invention

The present invention relates to:

(1) A polypeptide selected from the group consisting of:

① a polypeptide represented by an amino acid sequence of SEQ ID No.1 or No.2,
② a polypeptide comprising the polypeptide of ①,
③ a polypeptide having homology of at least about 70% to the polypeptide of ① in amino acid sequence and having an activity of decomposing phosphatidic acid, and
④ a polypeptide having mutation such as deletion, substitution, addition and insertion of one or a few amino acids in the above amino acid sequence and having an activity of decomposing phosphatidic acid;

(2) A peptide having at least about eight consecutive amino acids in the amino acid sequence of SEQ ID No.1 or No.2;

(3) A polynucleotide encoding the polypeptide or peptide of the above item 1 or 2, or its complementary chain;

(4) A polynucleotide which hybridizes with the polynucleotide or its complementary chain of the above item 3 under a selective condition;

(5) A polynucleotide having at least fifteen consecutive bases in the base sequence of SEQ ID No.3 or No.4 or its complementary sequence;

(6) A recombinant vector having the polynucleotide of the above item 3 - 5;

(7) A transformant transformed with the recombinant vector of the above item 6;

(8) A method for the production of the polypeptide of peptide of the above item 1 or 2, comprising a step of culturing the transformant of the above item 7;

(9) An antibody which immunologically recognizes the polypeptide or peptide of the above item 1 or 2;

(10) An antibody of the above item 9, which inhibits the activity of decomposing phosphatidic acid;

(11) A method for the identification of a compound which inhibits or activates of activity of the polypeptide of the above item 1 through an interaction with said polypeptide, and/or a compound which inhibits or promotes expression of the polynucleotide of the above item 3 or 4 through an interaction with said polynucleotide, comprising the use of at least one of the polypeptide or peptide of The above item 1 or 2, the polynucleotide of one of the above items 3 to 5, the vector of the above item 6, the transformant of the above item 7, the antibody of the above item 9 or 10;

(12) A method for the identification of a compound which inhibits or activates activity of the polypeptide of the above item 1 through an interaction with said polypeptide, and/or a compound which inhibits or promotes expression of the polynucleotide of the above item 3 or 4 through an interaction with said polynucleotide, comprising contacting the compound to be screened with the polypeptide or polynucleotide under a condition which enables interaction between the compound and the polypeptide or polynucleotide, accessing the interaction (the interaction is related to a second component which may provide a detectable signal in response to the interaction between the compound and the polypeptide or polynucleotide), and detecting the presence, absence or change of the signal due to the interaction between the compound and the polypeptide or polynucleotide so as to determine whether the compound may activate or inhibit the activity through the interaction with the polypeptide or polynucleotide;

(13) A method for the identification of a compound which inhibits or activates activity or physiological function of the polypeptide of the above item 1 or the polynucleotide of the above item 3 or 4, contacting the compound to be screened with the transformant of the above item 7 and another transformant expressing a receptor for lysophosphatidic acid that is produced by the function of the polypeptide of the above item 1 expressed in the transformant of the above item 7 on phosphatidic acid under a condition which enables interaction between the compound and these transformants, and accessing the interaction (the interaction is related to a second component that may provide a detectable signal in response to the interaction between the compound and the transformants), and detecting the presence, absence or change of the signal due to the interaction between the compound and the transformants so as to determine whether the compound may activate or inhibit the activity or physiological function of the polypeptide or polynucleotide;

(14) A compound identified by the method of the above item 11, 12 or 13;

(15) A compound which inhibits or activates activity of the polypeptide of the above item 1 through an interaction with said polypeptide, and/or a compound which inhibits or promotes expression of the polynucleotide of the above item 3 or 4 through an interaction with said polynucleotide;

(16) A pharmaceutical composition comprising at least one of the polypeptide or peptide of the above item 1 or 2, polynucleotide of the above item 3, 4 or 5, the vector of the above item 6, the transformant of the above item 7, the antibody of the above item 9 or 10, and the compound of the above item 14 or 15;

(17) A method for the diagnosis of a disease relating to the expression or activity of the polypeptide of the above item 1, comprising analyzing as a marker (a) a nucleic acid sequence encoding said polypeptide, and/or (b) the polypeptide in a sample derived from a subject;

(18) A method for the treatment of a phospholipase$A_1$-related disease comprising using the pharmaceutical composition of the above item 16; and

(19) A method for the production of the pharmaceutical composition of the above item 16.

Summarized Description of Drawings

Fig.1 shows the sequence and its features of the novel $PLA_1$ (short-type). A double line, an underline, and a line with arrows represent a signal sequence, a predicted sugar chain-added site, and a lipase consensus sequence and Lid region, respectively. "S", "D" and "H" surrounded by a square are an active triad.

Fig.2 is a continued part of Fig.1. A double line, an underline, and a line with arrows represent a signal sequence, a predicted sugar chain-added site, and a lipase consensus sequence and Lid region, respectively. "S", "D" and "H" surrounded by a square are an active triad.

Fig.3 illustrates a bio-system for studying the function of the novel $PLA_1$ using a cell expressing the novel $PLA_1$ and a cell having taken in Fura2 and expressing a LPA receptor, EDG7.

Fig.4 (A) demonstrates that the Sf9 expressing the novel $PLA_1$ increases an intracellular Ca2+ concentration in the Sf9 expressing the LPA receptor, EDG7, and that PLD is involved in the production of LPA catalyzed by the novel $PLA_1$.

Fig.4 (B) shows a concentration-depending function of 1-oleyl-LPA in the EDG7-expressing cells.

Best Mode for Carrying Out the Invention

(Novel $PLA_1$)

A cDNA of the novel $PLA_1$ provided by the present invention was obtained as a substance encoding a novel amino acid sequence from a cDNA library. The presence of the novel $PLA_1$ of the present invention was confirmed in human testis by a northern blotting. The novel $PLA_1$ of the present invention has the following features. It interacts with phospholipids, especially phosphatidic acid (PA) to generate LPA. It has a consensus sequence conserved in a lipase family and amino acid sequences which are considered to be a catalytic triad and Lid. It has homology of less than 40% to known $PLA_1$s.

(Polypeptide or Peptide)

An amino acid sequence of the novel $PLA_1$ of the present invention is a polypeptide represented by SEQ ID No.1 or No.2. Furthermore, the polypeptide or peptide of the present invention may be selected from a polypeptide or peptide having at least a part of the polypeptide represented by SEQ ID No.1 or No.2. The polypeptide or peptide thus selected has homology of at least about 40%, preferably at least about 70%, more preferably at least about 80%, still further preferably at least about 90%, and most preferably at least about 95% in amino acid sequence. The polypeptide or peptide having the above homology may be selected based on the presence of their activity of decomposing phospholipids, especially PA and/or on their substrate specificity for PA. The decomposing activity may be determined by known methods such as those using substances labeled with a radio isotope (RI) substrate, a fluorescence substrate or a coloring substrate, or those described in Examples of the present specification (J. Biochem, 103, 442-447, 1988; J. Biochem., 117, 1280-1287, 1995; J. Biochem., 101, 53-61, 1987; J. Biol. Chem., 235, 2595-2599, 1960; J. Biol. Chem., 272, 2192-2198, 1997).

Techniques for determining homology of the amino acid sequences are known by themselves, such as, for example, a method for directly determining them, and a method for predicting them based on a determined base sequence of cDNA.

The polypeptide or peptide of the present invention, which may include polypeptide or peptide having a partial sequence of the polypeptide represented by SEQ ID No.1 or No.2, may be used as an agent, a standard material or an immunogen. Thus, the minimum unit of the polypeptide or peptide of the present invention consists of a consecutive amino acid sequence of 8 or more amino acids, preferably 10 or more amino acids, more preferably 12 or more amino acids, still further preferably 15 or more amino acids. Preferably, said minimum unit of the polypeptide or peptide of the present invention can be immunologically detectable. These peptides may be used as the agent and standard material, and they may be also used as an antigen for the preparation of an antibody against the novel $PLA_1$ alone or in combination with carriers such as keyhole limpet hemocyanin or ovoalbumin. These complex compounds are also included in the present invention.

Furthermore, as being based on the presence of their activity of decomposing phospholipids, especially PA and/or their substrate specificity for PA, a polypeptide or peptide having mutation such as deletion, substitution, addition and insertion of one or more, for example, 1～100, preferably 1～30, more preferably 1～20, still further preferably 1～10, most preferably one or a few amino acids in the above amino acid sequence is provided by the present invention as well. The means for making the deletion, substitution, addition and insertion are known per se, including, for example, site specific mutation technique, recombination technique, primer extension method, polymerase chain reaction and any combination thereof, and those described in the text books such as Sambrook, et al., Molecular Cloning, A Laboratory Manual 2nd. Ed., Cold Spring Harbor Laboratory, 1989; Labomanual Gene Engineering, Maruzen Co., Ltd., 1988: Ehrlich HE., Ed, PCR Technology, Principle and Application of DNA amplification, Stockton Press, 1989 or any modification thereof such as that of Ulmer (Science, 219, 666, 1983).

In the process of the above mutation process, it is easily supposed that amino acids may be exchanged between those belonging to the same group such as a polar amino acid, a non-polar amino acid, a hydrophobic amino acid, a hydrophilic amino acid, a positively charged amino acid, a negatively charged amino acid, and an aromatic amino acid group. As the consensus sequence and Lid region of the lipase family are considered to be essential for expression or control of their activities as mentioned below, it is preferable to maintain a region containing the above sequence or region, especially the consensus region containing the catalytic triad in primary sequence and/or steric structure so that $PLA_1$ activity, especially PA-$PLA_1$ activity should be maintained. Although the polypeptide or peptide of the present invention may be glycosylated or not, at least one glycosylation site is preferably kept since the presence of a sugar chain may affect the activity.

The present invention also provides a polypeptide that has the same $PLA_1$ activity as that of the polypeptide represented by an amino acid sequence of SEQ ID No.1 or No.2, or its minimum active unit (region or domain), and a polypeptide whose strength of activity or substrate specificity have been modified. These substances are useful as a $PLA_1$ activity-like substance or an antagonist against $PLA_1$. They are also useful in screening a substance which controls $PLA_1$ activity. Any genetic material that is homologous to the present invention, which is derived from animals other than human, is also included in the scope of the sent invention.

It is easy for those skilled in the art that other proteins such as alkaline phosphatase, β-galactosidase, and Fc fragment of immunoglobulin such as IgG, and a peptide such as FLAG-tag may be linked directly or indirectly, for example, via a linker peptide to N- or C-terminal of the present polypeptide and the like in order to facilitate the detection or purification thereof. Accordingly, such compounds as conjugated with the above other substances are also included in the scope of the present invention.

(Polynucleotide)

In one aspect, the polynucleotide and its complementary chain of the present invention means that encodes the amino acid sequence of the polypeptide or peptide of the present invention, for example, that of SEQ ID No.1 or No.2, or its complementary chain. These polynucleotides provide useful genetic information for the production of the novel $PLA_1$. They will be also utilized as a nucleic acid agent or a standard material. In SEQ ID No.3 and No.4 that show preferable base sequences, it is estimated that a coding region extends from adenine (A) at base No.115 to adenine (A) at base No.1494, and from adenine (A) at base No.11 to adenine (A) at base No.1453, respectively. Also, a region (atg~gtg) encoding a peptide from Met (M) at amino acid No.1 to Val (V) at amino acid No.13 in SEQ ID No. 1 is estimated to encode a signal sequence.

In another aspect, the present invention provides a polynucleotide which hybridizes with the polynucleotide encoding the amino acid sequence of the polypeptide or peptide of the present invention such as that represented by the amino acid sequence of SEQ ID No.1 or No.2, preferably with a polynucleotide shown by the base sequence of SEQ ID No.3 or No.4, or in its complementary chain under a selective condition, preferably under a stringent condition. Hybridization may be performed in accordance with a method described in Sambrook, et al., Molecular Cloning, A Laboratory Manual 2nd. Ed., Cold Spring Harbor Laboratory, 1989; or in Shaw, et al., Nucleic Acids Res., Vol.11, 555-573, 1983, or any modification thereof. The phrase "selective condition" in the present specification means a condition under which a nucleic acid having a desired homology to a desired nucleic acid such as a specific probe (for example, the nucleic acid of SEQ ID No.3 encoding the present $PLA_1$) is selectively or specifically hybridized, while other non-related nucleic acids such as that having less degree of homology is not hybridized. A melting temperature (Tm) is generally used as a stability index for a double chain molecule (hybrid) of nucleic acids. Since Tm depends on length of the chain, base composition, and chemical compositions (ionic strength, the presence of a denaturing agent, etc.), hybridization is usually carried out below Tm. An equation of the value of Tm for a hybrid with a complete complementarity for DNA, RNA or oligonucleotide is experimentally obtained (Human Molecular Genetics, Tom Strachan and Andrew P. Read; Masami Muramatsu, Medical Sciences International, 1997). When a prove consists of less than 50 nucleotides, an estimated Tm value is obtained by the equation: Tm(°C)=4(G +C)+2(A+T), wherein A, T, G, C mean the numbers of each base in the probe. In order to selectively obtain the complete complementarity, the hybridization temperature should be set at 5°C below Tm or more (preferably at Tm or less). In order to maintain stability of the hybrid, the hybridization temperature needs to be lowered by 5°C for one unpaired base. On the other hand, when a prove consists of 50 nucleotides or more, the equation will be: Tm(°C)=81.5°C+16.6logM+0.41(%G+%C)-500/n-0.61 (% formamide) wherein "M" is ionic strength of monovalent cationic ions in a solution (mol/L), "n" is the number of base pairs in a double chain. As the hybridization temperature decreases by 1°C for one unpaired base, the hybridization temperature needs to be adjusted accordingly. The nucleic acid having a desired homology will be selectively hybridized by setting a hybridization temperature at 55°C below, preferably 40°C below, more preferably 25°C below, still further preferably 10°C below, most preferably 5°C below the Tm of the hybrid with the complete complementarity.

There is a method which has been partly modified based on Shaw et al. Specifically, a filter such as a nylon membrane or nitrocellulose filter, to which nucleic acids have been bound, is washed with 3 x SSC (Standard Saline Citrate; 1 X SSC:0.15M NaCl, 0.015M Sodium citrate) containing 0.1% SDS overnight at 65°C, and subjected to a prehybridization in 5 X SSCP (1 X SSCP:0.15M NaCl, 0.015M Sodium citrate, 10mM $NaH_2PO_4$, 1mM EDTA, pH7.2) containing 50% formamide, 5 Denhardt's solution, 0.1% SDS, 250 μg/ml denatured salmon sperm DNA for 5 hours at 42°C (or for 2 hours at 65°C). A suitable amount of RI-labeled or non RI-labeled probe is added to 5 X SSCP containing 50% formamide, 1 X Denhardt's solution, 0.1% SDS, 100 μg/ml denatured salmon sperm DNA and 10%(w/v) dextran sulfate, and then said filter is hybridized in the resulting mixture for 18 hours at 28°C, preferably at 37°C, more preferably at 42°C, still further preferably at 50°C, most preferably at 65°C. The filter is washed twice for 5 minutes each with 2 X SSCP containing 0.1% SDS at a room temperature, preferably at 37°C, and then with 0.3 X SSCP containing 0.1% SDS three times for one hour in total at 37°C, preferably at 50°C, more preferably at 65°C. The presence of the probe is then detected specifically by a suitable means such as autoradiography. The conditions for hybridization, washing and the like may be optionally combined depending on the kind of probe and the like. The above polynucleotide is not necessarily a complementary sequence as long as it can hybridize with the desired polynucleotide, especially with that shown by the base sequence of SEQ ID No.3 or No.4 or its complementary sequence. Thus, the polynucleotide has homology of at least about 40%, for example at least about 70%, preferably at least about 80%, more preferably at least about 90%, most preferably at least about 95% to the base sequence of SEQ ID No.3 or No.4 or its complementary sequence. The nucleotide of the present invention also includes a polynucleotide, oligonucleotide or their complementary chain corresponding to a designated region in the base sequence and consisting of a successive sequence of 10 or more nucleotides, preferably 15 or more nucleotides, more preferably 20 or more nucleotides.

These polynucleotides is useful as a probe or primer for the detection of the nucleic acid encoding the present novel $PLA_1$ such as its gene or mRNA in the production of polypeptide of the present invention, or useful as an antisense oligonucleotide used in the control of the gene expression. In this context, the polynucleotide includes not only one encoding a translation region but also one encoding a non-translation region. For example, it may be conceived that a specific base sequence in the novel $PLA_1$ which is different from a consensus sequence region conserved in the lipase family is used to specifically inhibit the expression of the novel $PLA_1$ with the antisense sequence. On the other hand, it may be possible to simultaneously inhibit several kinds of the lipases including the novel $PLA_1$ by using the conserved sequence. The base sequence encoding the novel $PLA_1$ and the polypeptide having the similar activity may be determined by, for example, confirming a protein expressed in a known protein expression system, and selecting it using as an index its physiological activity, especially the activity of decomposing phosphatidic acid. When the protein is expressed in a cell-free system, techniques of a ribosome system derived from embryo, rabbit reticulocyte and the like may be used (Nature, 179, 160-161, 1957).

(Transformant)

The novel $PLA_1$ and the polypeptide peptide derived therefrom may be provided by means of gene recombination techniques using known host cells such as E.coli, yeast, bacillus, insect cells, and animal cells. Although an insect cell was used in the example of the present invention, the scope of the present invention will not be limited thereto without doubt (Japanese Patents No.2129487 and No. 2644447: A method for the preparation of baculovirus expression vector and synthesis of polypeptide). As the $PLA_1$ encoded by the present novel gene is a glycoprotein, it is preferable to use as the host cells that can add a sugar chain to the protein, such as animal cells.

Transformation may be carried out by any known method such as those using plasmid, chromosome, virus and the like as a replicon. Integration of a gene into the chromosome is a preferable method in view of its stability. An auto-replication system using an extranuclear gene may be conveniently utilized. A vector, which comprises as its constituents a gene sequence to be expressed and a gene sequence having information for replication and control, is selected depending on the host cell to be used. Such constituents may be selected and combined from a promoter, a ribosome-binding site, a terminator, a signal sequence, an enhancer and the like with known methods depending on procaryotic or eucaryotic cells. Although a baculovirus system was used in the example of the present invention, the scope of the present invention will not be limited thereto without doubt.

The transformant may be cultured in the most preferable conditions for each host cell, which are known per se. The culture may be done on the basis of an enzymatic activity of the novel $PLA_1$ and the present polypeptide and peptide derived therefrom, especially that of decomposing phosphatidic acid as an index, or alternatively, it may be done continuously or batch wise using the amount of the transformant in a culture medium as an index.

(Collection of novel $PLA_1$ and other material derived therefrom)

The novel $PLA_1$ and the peptide and polypeptide derived therefrom may be collected and purified on the basis of the activity of decomposing phosphatidic acid as an index, by means of molecular sieve, ion chromatography, affinity chromatography, and any combination thereof, or any fractionation method such as that based on difference in solubility with ammonium sulfate and alcohol. Preferably, based on the information about the amino acid sequence of the present invention, a polyclonal or monoclonal antibody against it may be prepared and used in specifically absorbing and collecting the above materials.

(Antibody)

An antigen determinant region of the novel $PLA_1$ and polypeptide and peptide derived therefrom is selected and prepared. The antigen may be the novel $PLA_1$ itself, or any fragment thereof consisting of the amino acid sequence of 8 or more amino acids, preferably 10 or more amino acids, more preferably 12 or more amino acids, still further preferably 15 or more amino acids. In order to prepare an antibody specific for the novel $PLA_1$, it is preferable to use the base sequence specific for the novel $PLA_1$, which is different from the consensus sequence region conserved in the lipase family. This amino acid sequence is not necessarily identical to that of SEQ ID No.1 or No.2. The above specific sequence is preferably located at a part of the protein steric structure, which is exposing outwards. When the exposing part is formed with non-sequential amino acids, the amino acid sequence used as the antigen may efficiently consist of these sequential amino acids relating to the exposing part. The present antibody includes any antibody as long as it binds to or recognizes the novel $PLA_1$ and the polypeptide or peptide derived therefrom. The presence or absence of such binding or recognition may be determined by a known antigen-antibody reaction.

For the preparation of the antibody of the present invention, humoral or cellular immune response is induced in animals by means of the novel $PLA_1$ and polypeptide or peptide derived therefrom alone or in conjugation with a carrier under the existence or non-existence of an adjuvant. Any carrier may be uses as long as it will not cause any disadvantages in the host, including cellulose, polymerized amino acids, albumin and the like. The animals to be immunized are preferably mouse, rat, rabbit, goat and horse. The polyclonal antibody may be collected from antiserum with a known method such as immune affinity chromatography.

The monoclonal antibody may be prepared by collecting an antibody-producing cells such as spleen cells and lymphocytes from the immunized animals, being fused with immortalized cells, for example, myelomas such as P3X63Ag8 strain to give hybridomas, cloning the resulting hybridomas, selecting a hybridoma which produces an antibody specifically recognizing the present $PLA_1$ and collecting the antibody from a culture medium of the hybridoma.

The polyclonal or monoclonal antibody of the present invention can inhibit the $PLA_1$ activity by directly bonding to the novel $PLA_1$ the present invention and controlling its activity so that LPA production system from phospholipid, especially PA may be easily controlled. Accordingly, these antibodies are useful in the treatment and/or prevention of various deteriorating diseases in which LPA is involved.

(Identification and screening of the compound)

The thus prepared novel $PLA_1$ and polypeptide or peptide derived therefrom, polynucleotide and its complementary chain, cells transformed on the basis of the information on the amino acid and base sequences, protein-producing system using these materials, and antibody recognizing the novel $PLA_1$ and polypeptide or peptide derived therefrom may provide a useful means in the identification or screening method of a controlling, inhibiting or increasing agent or material of the activity of the novel $PLA_1$ and polypeptide or peptide derived therefrom and polynucleotide. For example, it is possible to utilize the selection of antagonist through the drug-design based on the steric structure of the peptide of polypeptide, the selection of an expression-controlling agent in a gene level by means of the protein-producing system, the selection of a material recognized by the antibodies and the like in a known screening system of pharmaceutical products. The term "controlling" means inhibiting, antagonizing, activating, activity-promoting, activity-increasing and the like.

The thus prepared novel $PLA_1$ and polypeptide or peptide derived therefrom, polynucleotide and the transformant may be used to select the conditions for enabling an interaction between the compounds to be screened and the polypeptide or peptide, introduce a system in which a signal (or marker) that can detect the presence or absence of the above interaction, and detect the presence, absence or change of the amount of the signal or marker, so that it is possible to identify a compound which inhibits or activates the activity of the novel $PLA_1$ and polypeptide or peptide derived therefrom or a compound which inhibits or promotes the expression of the present polynucleotide. The system using the signal or marker includes a system for determining the activity of the present polypeptide, for example, that of decomposing the substrate such as PA, or a system for determining the amount of the expression of the polynucleotide, some of which are described in Examples of the present specification. Any known methods may be applied to these systems.

A compound which activates or inhibits the activity or physiological function of the novel $PLA_1$ and polypeptide derived therefrom or the polynucleotide of the present invention is identified by contacting the compound to be screened with the transformant expressing the novel $PLA_1$ and polypeptide derived therefrom and another transformant expressing a receptor for lysophosphatidic acid that is produced by the function of the novel $PLA_1$ expressed in said transformant and polypeptide derived therefrom on phosphatidic acid under a condition which enables the interaction between the compound and these transformants or polypeptide, and detecting the presence, absence or change of the signal. As non-limiting examples of the above transformants there may be mentioned Sf9 cell expressing the novel $PLA_1$ and polypeptide derived therefrom and Sf9 cell expressing LPA receptor, EDG7. The signal used in the detection of the function of the novel $PLA_1$ and polypeptide derived therefrom includes an amount of intracellular calcium, which will be increased in response to the binding of LPA to the cell expressing LPA receptor, EDG7. The amount of intracellular calcium may be detected by utilizing a known method using, for example, Fura2. Furthermore, the specificity of the function of the compound may be confirmed by comparing the above reaction with a reaction in which the present polypeptide is replaced with another lipase equivalent or polypeptide or LPA. Each transformant may be replaced with the other cell strains that have been confirmed to express the corresponding genes.

(Compound, Pharmaceutical Compound),

The thus identified compounds are utilized as a candidate of an inhibitor, antagonist, activator, promoter or potentiator for the novel $PLA_1$ and polypeptide or peptide derived therefrom. They are also useful as a candidate of an inhibitor, antagonist, activator, promoter or potentiator for the expression of the novel $PLA_1$ and polypeptide or peptide derived therefrom. Accordingly, these compounds are expected to be used in the treatment and/or prevention of various deteriorating diseases in which LPA is involved.

The thus identified compounds may be then selected in view of their biological utility and toxicity and formulated as a pharmaceutical composition. The novel $PLA_1$ and polypeptide or peptide derived therefrom, the polynucleotides encoding them, and their complementary chains, the vectors having their base sequences, and the antibodies immunologically recognizing the novel $PLA_1$ and polypeptide or peptide derived therefrom may be used per se as a diagnosis means such as a marker and an agent. They may be also used as a pharmaceutical means such as a treating medicine based on their activities of inhibiting, antagonizing, activating, promoting and potentiating the expression, activity or function of the novel $PLA_1$. Known formulation methods may be introduced depending on the peptide or polypeptide, protein, polynucleotide, antibody and the like. The present pharmaceutical composition is prepared by using the novel $PLA_1$ and polypeptide or peptide derived therefrom, the polynucleotide, the vector, the transformant, the antibody and the compound of the present invention. The above pharmaceutical composition is useful in the treatment of the novel $PLA_1$-relating diseases.

The present pharmaceutical composition is useful as a diagnosis means of the diseases relating to the expression or activity of the novel $PLA_1$ and polypeptide or peptide derived therefrom. The diagnosis may be performed by determining the amount of a nucleic acid sequence corresponding to the nucleic acid sequence encoding the above peptide by means of their interaction or reactivity with said nucleic acid sequence and/or determining a distribution in a living body of the above peptide, and/or determining the existence of the above peptide, or an amount or degree of the activity of the above peptide contained in a sample derived from an individual, and the like. Thus, the novel $PLA_1$ is assayed as a diagnosis marker. The method for the determination is a known one such as antigen-antibody reaction, enzyme reaction, PCR reaction and the like. Assay of single nucleotide polymorphism (SNP) by means of a known method is also a useful diagnosis means.

Examples

The present invention will now be explained by referring to the Examples, which will not limit the scope of present invention.

(Isolation of gene)

Homology search (tblastn search) was done on dbEST (database of Expressed Sequence Tags) using as a probe an amino acid sequence of rat phospholipase A1 (PS-PLA1) that specifically hydrolyze phosphatidic serine (J. Biol.Chem.; 272, (4),2192-2198, 1997). As a result, an EST sequence (Accession No.AA470035) with an unidentified sequence was obtained as one having a relatively high homology score.

The next homology search (blastn search) was done on GenBank using as a probe the base sequence of Accession No.AA470035. As a result, the sequences of Accession No.AP006556 and Accession No.AP001347 were picked up. Primers were then designed based on these sequences, and PCR reaction was carried out using the primers and a first strand that had been prepared from human testis total RNA with an oligo dT primer as a template. As it was difficult to determine the sequence at 5'end, it was confirmed by 5'-RACE method using Primer A (SEQ ID No.5: ATTTTGTTCAAACAGTGGCTCAGCA), Primer B (SEQ ID No.6:TTCAAACAGTGGCTCAGCACAGTTT) and Marathon-Ready™ cDNA Human Testis (Clontech). As a result, the sequences of two isoforms ("short-type" and "long-type") were found, which were different in the length of a first exon. These two sequences were then aligned and characterized. It was estimated that there existed the features characteristic to the PS-PLA1 and lipase such as amino acid residues of an active triad and a loop structure called "Lid" in the steric proximity of an active site pocket (B.A.A., 1376, 417-432, 1998; Biochemistry, 32, 4702-4707, 1993 Protein, Nucleic Acid and Enzyme, 44, 1038-1042, 1999), suggesting the possibility that the above sequences were novel phospholipase A1.

(Cloning of Novel Sequence)

For the purpose of cloning cDNA having the DNA sequence of the novel PLA1 as estimated above, RT-PCR was performed on RNA from Human Testis (Clontech) using a mixture of the oligonucleotides of a forward primer: Primer C (SEQ ID No.7: 5'-CGCGGATCCATGTTGCTCAAATGTTTACATAAT-3') or Primer D (SEQ ID No.8:5'-CGCGGATCCATGAGAGTATACATTTTTCTTTGT-3'), and a reverse primer: Primer E (SEQ ID No.9:5'-AAATATGCGGCCGCTTATGTGTTCTTTGGTGTACATGT-3'). The amplified two kinds of gene fragments (ca. 1.7kbp) were inserted separately into BamHI/NotI restriction site in a multicloning site of pFASTBac1 (Lifetech Oriental Co.) and transfected into E.coli JM109. Positive clones were selected and subjected to cloning. The vectors were collected and the base sequences were determined by a conventional method. The plasmid containing a coding region (short-type) of the base sequence represented by SEQ ID No.3, pFASTBac-papla1β) was deposited with the International Patent Organism Depository, the National Institute of Advanced Industrial Science and Technology (the former name of the National Institute of Bioscience and Human technology, Agency of Industrial Science and Technology)(1-3, Higashi 1-chome, Tsukuba-shi, Ibaraki-ken 305-8566 JAPAN) on October 5, 2000 under Accession No.FERM P-18072, and then transferred on August 8, 2001 to the deposit under the terms of the Budapest Treaty on the International Recognition of the Deposit of Microorganisms for the Purposes of Patent Procedure and Regulation under Accession No.FERM BP-7697.

The cDNAs represented by SEQ ID No.3 (short-type) and No.4 (long-type) contain an open reading frame of 1380 base pairs encoding 460 amino acids (SEQ ID No.1) and 1443 base pairs encoding 481 amino acids (SEQ ID No.2), respectively. At least SEQ ID No.3 (short-type) contained a region of a supposedly signal sequence at its N-terminal region. As the characteristics in the amino acid sequences, a motif of asparagine glycosylation site: N-{P} -[ST]-{P} was found at two site in both the short- and long-types, i.e., N(Asn)63-L(Leu)66 and N(Asn)396-S(Ser)399, and N(Asn)84-L(Leu)87 and N(Asn)417-S(Ser)420, respectively.

(Homology to known proteins)

The homology search (tblastn search) was done on GenBank using as a probe an amino acid sequence estimated from the translation of the above base sequences. As a result, the present novel $PLA_1$ (colon lipase) showed a significantly high degree of homology to human PS- $PLA_1$ (hPS- $PLA_1$), human pancreatic lipase, lipoprotein lipase, plrp1 (pancreatic lipase related protein 1) and plrp2 (pancreatic lipase related protein 2). They also showed a high homology to vitellogenin that was thought to have a region with a relatively high degree of steric homology to lipase. As it was confirmed that the amino acid sequence estimated to be the enzymatic activity triad (S(Ser)159, D(Asp)183 and H(His)253 for the short-type, and S(Ser180, D(Asp)204 and H(His)274 for the long-type) was conserved in all of these known proteins having the high degree of homology except vitellogenin, their multiple alignment was analyzed using GENETYX Multiple Alignment module (Software Developing Co.,).

As a result, it was found that there existed the consensus sequences conserved in the lipase family, i.e., GXSXG ((Gly)157-G(Gly)161 and G(Gly)178-G(Gly)182), ITGLD(I(Ile)179-D(Asp)183 and I(Ile)200-D(Asp)204), and CXH(C(Cys)251-H(His)253 and C(Cys)272-H(His)274) wherein "X" means an arbitrary amino acid and that these consensus sequences contained all of the amino acid residues that were considered to constitute the enzymatically active triad. Furthermore, it was revealed that the loop structures (P(Pro)239-K(Lys)250 and P(Pro)260-K(Lys)271), which control the expression of the lipase activity and are called "Lid", of the same number as that of PS-$PLA_1$, i.e., 12 loop structures existed in the steric proximity of a pocket containing the active triad. It has been shown that lipases other than PS- $PLA_1$ have longer amino acids residues in the Lid structure, and that their activity will be effected upon biding of a proteinous factor called "co-lipase" to them (B.B.A., 1376, 417-432, 1998; Biochemistry, 32,4702-4707, 1993; Protein, Nucleic Acid and Enzyme, 44, 1038-1042, 1999). However, it has not yet been demonstrated that PS-$PLA_1$ having a relatively short Lid needs co-lipase. Accordingly, it is estimated that the protein translated from the resulting base sequence may possibly show its activity in a similar mechanism to that of PS-$PLA_1$.

Evolutionary phylogenetic tree was estimated about PLA1 lipase family using GENETYX Evolutionary tree (UPGMA method) module (Software Developing Co.). The results showed that the novel sequence is the evolutionarily nearest one to PS-$PLA_1$. Accordingly, the protein translated from the novel sequence has been estimated to a novel lipase which closely relates to lipases, particularly to phospholipase.

(Confirmation of Expression in Tissue)

Northern blotting was performed using human normal tissues in order to examine expression of the novel PS-$PLA_1$ in the tissues. A cDNA fragment with about 0.5 kbp within an open reading frame of the above sequence was used as a probe. Thus, a forward primer of the oligonucleotide consisting of the base sequence (SEQ ID No.10: 5'-AAAAACACCAGAAAAGTTGCTGTGAG-3') which corresponds to the base No.493-518 in SEQ ID No.3, and a reverse primer of the oligonucleotide consisting of the base sequence ((SEQ ID No.11:5'-GCTTGATAACCCAGCCGAGGACATG-3') which corresponds to the complementary chain of the base No.1001-1025 in SEQ ID No.3 were synthesized and a $^{32}P$-labelled probe was prepared by PCR using the above primers. The northern blotting was done according to User Manual (PT1200-1, Clontech) by means of Human Multiple Tissue Northern Blot (Clontech). The results showed that a high expression of mRNA was observed in testis among the normal tissues examined (heart, brain, placenta, lung, liver, skeletal muscle, pancreas, kidney, thymus, prostate, testis, ovary, small bowel, colon, and leukocyte).

(Relation with LPA Receptor)

Because the novel $PLA_1$ of the present invention showed the highest homology (about 45%) in amino acid sequence to the PLA1 that hydrolyzes PA to give 2-acyl LPA (the Japanese Patent Application Laid Open Hei.11 (1999)-187089), it is considered that the novel $PLA_1$ of the present invention may possibly show the same activity and therefore produce and provide LPA as a ligand with a LPA receptor. The known LPA receptors include EDG2, EDG4 and EDG7. Among them EDG7 is such a unique receptor that it has a strong reactivity with LPA having unsaturated fatty acids and reacts more strongly with 2-acyl-LPA than with 1-acyl-LPA, whose ligand specificity is different form that of EDG2 and EDG4 (J. Biol. Chem., 274, pp.27776-27785, 1999). As it is expected that any $PLA_1$ reaction has to be involved in a signal transmission through EDG7, the expression of the novel $PLA_1$ and EDG7 in organic tissues was examined by the northern blotting, giving the result that both of their mRNAs were expressed well in testis.

Next, the possibility was examined that the novel $PLA_1$ may hydrolyze PA to give LPA as a ligand for EDG7 by means of a bioassay system as shown in Fig.3. The insect cell "Sf9" (derived from ovary tissue of the pupa of Spodoptera frugiperda) , which lacked in the reactivity with LPA, was used in the bioassay. The novel $PLA_1$ was expressed in the above cell by means of a baculovirus system (this will be referred to also as an "enzyme party"). Thus, DH10BACT$^M$ competent cell (GIBCO BRL) was transfected with the recombinant pFASTBac plasmid cloned in the above example and a recombinant Bacmid was collected. Sf9 cell was then transfected with the resulting Bacmid and CellFECTIN™ to give a recombinant Baculovirus in a supernatant of the culture medium. The Sf9 cell that expresses the novel $PLA_1$ was obtained by infecting Sf9 cell with the recombinant Baculovirus. On the other hand, the LPA receptor, EDG7 was expressed by Sf9 cell by using the Baculovirus system according to the method of J. Biol. Chem., 274, pp. 27776-27785, 1999(this will be referred to also as a "receptor party"). In this system, if a sufficient amount of the novel $PLA_1$ is expressed to produce LPA, the binding of LPA to the LPA receptor-expressing cell will induce the signal transmission in the cell and increase the intracellular concentration of $Ca^{2+}$. Accordingly, the production and function of the novel $PLA_1$ can be examined by change of the $Ca^{2+}$ concentration. The change of the $Ca^{2+}$ concentration was determined with an indicator of $Ca^{2+}$ fluorescence, Fura-2. The LPA receptor-expressing Sf9 cell was suspended into a nutritional solution for Sf9 Ca-assay (10mM $CaCl_2$, 60mM KCl, 17mM $MgCl_2$, 10mM NaCl, 10mM MES, 4mM glucose, 110mM sucrose, 0.1% bovine serum albumin) at the concentration of 5 x $10^5$ cells/ ml, and 2μM Fura2-AM was incorporated into the cells for one hour at 27°C. After being washed twice with the above nutritional solution, the cell was re-suspended into the nutritional solution at the concentration of 5 x $10^5$ cells/ ml. The PLA1-expressing Sf9 cell was suspended into the nutritional solution at the concentration of 5 x $10^5$ cells/50μl and cultured for 30 min. After one ml of the LPA receptor-expressing cell thus prepared was taken into a cuvet, the cell was exposed to excitation spectrum at 340 nm and 380 nm with stirring by means of a micro-stirrer, and intensity of fluorescence emitted at 500 nm in response to each excitation and a ratio between their intensity were measured by means of CAF- 210 type Intracellular Ion detector (Nihon Optics Industry Co.). The same measurement was repeated after adding the PLA1-expressing Sf9 cell to the cuvet. Further, the values were obtained also in the case that all of the Fura2 was conjugated with extracellular $Ca^{2+}$ due to the addition of Triton-X100 at the time of measuring, and in the case that all of the Fura2 was dissociated from extracellular $Ca^{2+}$ due to the chelation with added EGTA at the time of measuring. Based on the following equation, the intracellular $Ca^{2+}$ concentration was calculated:

$$[Ca^{2+}](nM) = 224 \times b/a \times (F-Fmin)/(Fmax-F)$$

In the above equation, the number "224" is a dissociation constant of Fura2; "a" is the fluorescence intensity in response to the emission at 380 nm in the case that all of the Fura2 was conjugated with extracellular $Ca^{2+}$ due to the addition of Triton-X100; "b" is the fluorescence intensity in response to the emission at 380 nm in the case that all of the Fura2 was dissociated with extracellular $Ca^{2+}$ due to the addition of EGTA; "F" is the ratio ((the fluorescence in response to the emission at 340 nm)/ ( the fluorescence in response to the emission at 380 nm)); "Fmax" means the value of "F" in the case that all of the Fura2 was conjugated with extracellular $Ca^{2+}$ due to the addition of Triton-X100; and "Fmin" means the value of "F" in the case that all of the Fura2 was dissociated from extracellular $Ca^{2+}$ due to the chelation with added EGTA.

As a result, it was observed that the intracellular $Ca^{2+}$ concentration would be increased when the supernatant of the culture medium of the PLA1-expressing Sf9 cell was added to the LPA receptor-expressing Sf9 cell (Fig.4A). This phenomenon was not observed in the case that either of the receptor party and enzyme party was replaced by a cell infected with a wild type baculovirus. These results therefore suggest that the novel PLA1 hydrolyzes intrinsic PA in the cell to give LPA, which will interact with the LPA receptor-expressing cell.

(Participation of PLD in the production of LPA by the novel PLA1)

It is known that phospholipase D (PLD), which converts membrane phospholipids into PA, participates also in the production of LPA in ovary cancer. The PLA1-expressing Sf9 cell was treated with PLD derived from actinomyces and a supernatant of the culture medium was collected after 30 min. The LPA receptor-expressing Sf9 cell was treated with the supernatant and the intracellular $Ca^{2+}$ concentration was determined as the above. The result showed that the cell treated with PLD could induce the intracellular $Ca^{2+}$ response with a lower concentration than the untreated cell did. It was therefore conceived that the novel $PLA_1$ would produce LPA (possibly 2-acyl-1-lysoPA) in combination with the activation of PLD.

Industrial Applicability

The present invention provides the novel $PLA_1$ that belongs to the $PLA_1$ lipase family. The novel $PLA_1$ is a cell-bound glycoprotein having a ligand specificity for PA, which hydrolyzes PA to produce LPA. Based on the finding of the mechanism of production of LPA in the cell by the novel PA-specific lipase ($PLA_1$) and of transferring of LPA from the cell to the LPA receptor, EDG7, the present invention further provides clues for solving the physiological significance of the $PLA_1$ family and the mechanism of production of the ligand for the LPA receptor. A novel pharmaceutical composition and diagnosis method, which may be provided on the basis of the above findings, have a great utility in lipase-related clinical and basic medical fields.

**Claims**

**1.** A polypeptide selected from the group consisting of:

① A polypeptide represented by an amino acid sequence of SEQ ID No.1 or No.2;
② A polypeptide comprising the polypeptide of ① ;
③ A polypeptide having homplogy of at least about 70% to the polypeptide of ① in amino acid sequence and having an activity of decomposing phosphatidic acid; and
④ A polypeptide having mutation such as deletion, substitution, addition and insertion of one or a few amino acids in the above amino acid sequence and having an activity of decomposing phosphatidic acid.

**2.** A polypeptide having at least about eight consecutive amino acids in the amino acid sequence of SEQ ID No.1 or No.2.

**3.** A polynucleotide encoding the polypeptide or peptide of Claim 1 or 2, or its complementary chain.

**4.** A polynucleotide which hybridize with the polynucleotide or its complementary chain of Claim 3 under a selective condition.

**5.** A polynucleotide having at least fifteen consecutive bases in the base sequence of SEQ ID No.3 or No.4, or its complementary sequence.

**6.** A recombinant vector having the polynucleotide of Claim 3-5.

**7.** A transformant transformed with the recombinant vector of Claim 6.

**8.** A method for the production of the polypeptide of peptide of Claim 1 or 2, comprising a step of culturing the transformant of Claim 7.

**9.** An antibody which immunologically recognizes the polypeptide or peptide of Claim 1 or 2.

**10.** An antibody of Claim 9, which inhibits the activity of decomposing phosphatidic acid.

**11.** A method for the identification of a compound which activates or inhibits or inactivates the polypeptide of Claim 1 through an interaction with said polypeptide, and/or a compound which promotes or inhibits expression of the polynucleotide of Claim 3 or 4 through an interaction with said polynucleotide, comprising the use of at least one of the polypeptide or peptide of Claim 1 or 2, the polynucleotide of one of Claims 3 to 5, the vector of Claim 6, the transformant of Claim 7, the antibody of Claim 9 or 10.

**12.** A method for the identification of a compound which inhibits or activates activity of the polypeptide of Claim 1 through an interaction with said polypeptide, and/or a compound which inhibits or promotes expression of the polynucleotide of Claim 3 or 4 through an interaction with said polynucleotide, comprising contacting the compound to be screened with the polypeptide or polynucleotide under a condition which enables interaction between the compound and the polypeptide or polynucleotide, accessing the interaction (the interaction is related to a second component that may provide a detectable signal in response to the interaction between the compound and the polypeptide or polynucleotide), and detecting the presence, absence or change of the signal due to the interaction between the compound and the polypeptide or polynucleotide so as to determine whether the compound may activate or inhibit the activity through the interaction with the polypeptide or polynucleotide.

**13.** A method for the identification of a compound which inhibits or activates activity or physiological function of the polypeptide of Claim 1 or the polynucleotide of Claim 3 or 4, contacting the compound to be screened with the transformant of Claim 7 and another transformant expressing a receptor for lysophosphatidic acid that is produced by the function of the polypeptide of Claim 1 expressed in the transformant of Claim 7 on phosphatidic acid under a condition which enables interaction between the compound and these transformants, accessing the interaction (the interaction is related to a second component that may provide a detectable signal in response to the interaction between the compound and the transformants), and detecting the presence, absence or change of the signal due to the interaction between the compound and the transformants so as to determine whether the compound may inhibit or activate the activity or physiological function of the polypeptide or polynucleotide;.

**14.** A compound identified by the method of Claim 11, 12 or 13.

**15.** A compound which inhibits or activates activity of the polypeptide of Claim 1 through an interaction with said polypeptide, and/or a compound which inhibits or promotes expression of the polynucleotide of Claim 3 or 4 through an interaction with said polynucleotide.

**16.** A pharmaceutical composition comprising at least one of the polypeptide or peptide of Claim 1 or 2, polynucleotide of Claim 3, 4 or 5, the vector of Claim 6, the transformant of Claim 7, the antibody of Claim 9 or 10, and the compound of Claim 14 or 15.

**17.** A method for the diagnosis of a disease relating to the expression or activity of the polypeptide of Claim 1, comprising analyzing as a marker (a) nucleic acid sequence encoding said polypeptide, and/or (b) the polypeptide in a sample derived from a subject.

**18.** A method for the treatment of a phospholipaseA1-related disease comprising using the pharmaceutical composition of Claim 16.

**19.** A method for the production of the pharmaceutical composition of Claim 16.

```
        10         20         30         40         50         60
ttttacagaa gaacctgcca gcctgtgatg atcctaccaa agagaaacct caatgagtta
tggaatttcc tttttggtga attgagtgct gtttttgctt ttctcagatt ccaaATGAGA
                                                           M R
GTATACATTT TTCTTTGTTT GATGTGCTGG GTGAGATCTG ATAATAAAAG ACCATGCCTT
V  Y  I  F  L  C  L  M  C  W  V  R  S  D  N  K  R  P  C  L
GAATTCTCTC AGCTAAGTGT AAAGGATTCC TTCAGAGATT TATTTATTCC GAGAATAGAG
E  F  S  Q  L  S  V  K  D  S  F  R  D  L  F  I  P  R  I  E
ACCATTCTGA TGATGTATAC AAGGAACAAC CTAAACTGTG CTGAGCCACT GTTTGAACAA
T  I  L  M  M  Y  T  R  N  N  L  N  C  A  E  P  L  F  E  Q
AATAACTCAC TTAATGTTAA TTTCAACACA CAAAAGAAAA CAGTCTGGCT TATTCACGGA
N  N  S  L  N  V  N  F  N  T  Q  K  K  T  V  W  L  I  H  G
TACAGACCAG TAGGCTCCAT CCCATTATGG CTTCAGAACT TCGTAAGGAT TTTGCTGAAT
Y  R  P  V  G  S  I  P  L  W  L  Q  N  F  V  R  I  L  L  N
GAAGAAGATA TGAATGTAAT TGTAGTAGAC TGGAGCCGGG GTGCTACAAC TTTTATTTAT
E  E  D  M  N  V  I  V  V  D  W  S  R  G  A  T  T  F  I  Y
AATAGAGCAG TTAAAAACAC CAGAAAAGTT GCTGTGAGTT TGAGTGTGCA CATTAAAAAT
N  R  A  V  K  N  T  R  K  V  A  V  S  L  S  V  H  I  K  N
CTTTTGAAGC ATGGTGCATC TCTTGACAAT TTTCATTTCA TAGGTGTGAG TTTAGGGGCT
L  L  K  H  G  A  S  L  D  N  F  H  F  I  G  V [S] L  G  A
CATATCAGTG GATTTGTTGG AAAGATATTT CATGGTCAAC TTGGAAGAAT AACAGGTCTT
H  I  S  G  F  V  G  K  I  F  H  G  Q  L  G  R  I  T  G  L
GACCCTGCTG GGCCAAGGTT CTCCAGAAAA CCACCATATA GCAGATTAGA TTACACGGAT
[D] P  A  G  P  R  F  S  R  K  P  P  Y  S  R  L  D  Y  T  D
GCAAAGTTTG TGGATGTCAT CCATTCTGAC TCCAATGGTT TAGGCATTCA AGAGCCCTTG
A  K  F  V  D  V  I  H  S  D  S  N  G  L  G  I  Q  E  P  L
GGACATATAG ATTTTTATCC AAATGGAGGA AATAAACAAC CTGGCTGTCC TAAATCAATT
G  H  I  D  F  Y  P  N  G  G  N  K  Q  P  G  C  P  K  S  I
TTCTCAGGAA TTCAATTCAT TAAATGCAAC CACCAGAGAG CAGTTCACTT GTTCATGGCA
F  S  G  I  Q  F  I  K  C  N [H] Q  R  A  V  H  L  F  M  A
```

Fig.1

TCTTTAGAAA CAAACTGCAA TTTTATTTCA TTTCCTTGTC GTTCATACAA AGATTACAAG
S L E T N C N F I S F P C R S Y K D Y K
ACTAGCTTAT GTGTGGACTG TGACTGTTTT AAGGAAAAAT CATGTCCTCG GCTGGGTTAT
T S L C V D C D C F K E K S C P R L G Y
CAAGCCAAGC TATTTAAAGG TGTTTTAAAA GAAAGGATGG AAGGAAGACC TCTTAGGACC
Q A K L F K G V L K E R M E G R P L R T
ACTGTGTTTT TGGATACAAG TGGTACATAT CCATTCTGTA CCTATTATTT TGTTCTCAGT
T V F L D T S G T Y P F C T Y Y F V L S
ATAATTGTTC CAGATAAAAC TATGATGGAT GGCTCGTTTT CATTTAAATT ATTAAATCAG
I I V P D K T M M D G S F S F K L L N Q
CTTGGAATGA TTGAAGAGCC AAGGCTTTAT GAAAAGAACA AACCATTTTA TAAACTTCAA
L G M I E E P R L Y E K N K P F Y K L Q
GAAGTCAAGA TTCTTGCTCA ATTTTATAAT GACTTTGTAA ATATTTCAAG CATTGGTTTG
E V K I L A Q F Y N D F V N I S S I G L
ACATATTTCC AGAGCTCAAA TCTGCAGTGT TCCACATGCA CATACAAGAT CCAGAGACTC
T Y F Q S S N L Q C S T C T Y K I Q R L
ATGTTAAAAT CACTTACATA CCCAGAAAGA CCACCACTTT GCAGGTATAA TATTGTACTT
M L K S L T Y P E R P P L C R Y N I V L
AAAGACAGAG AGGAAGTGTT TCTTAATCCA AACACATGTA CACCAAAGAA CACATAAgat
K D R E E V F L N P N T C T P K N T *
gccttcttcc atcaaatgca cttgcttgtg aattaatgga cttgtaaatg aaacaatgca
atcagtcttt tataatgcac tgttcaattt gagattcaag tatttctatt tcttggaaaa
aattttaaga atcaaaaata aagaaaataa aaaatgcata cagttaaaca ttccaaa

Fig. 2

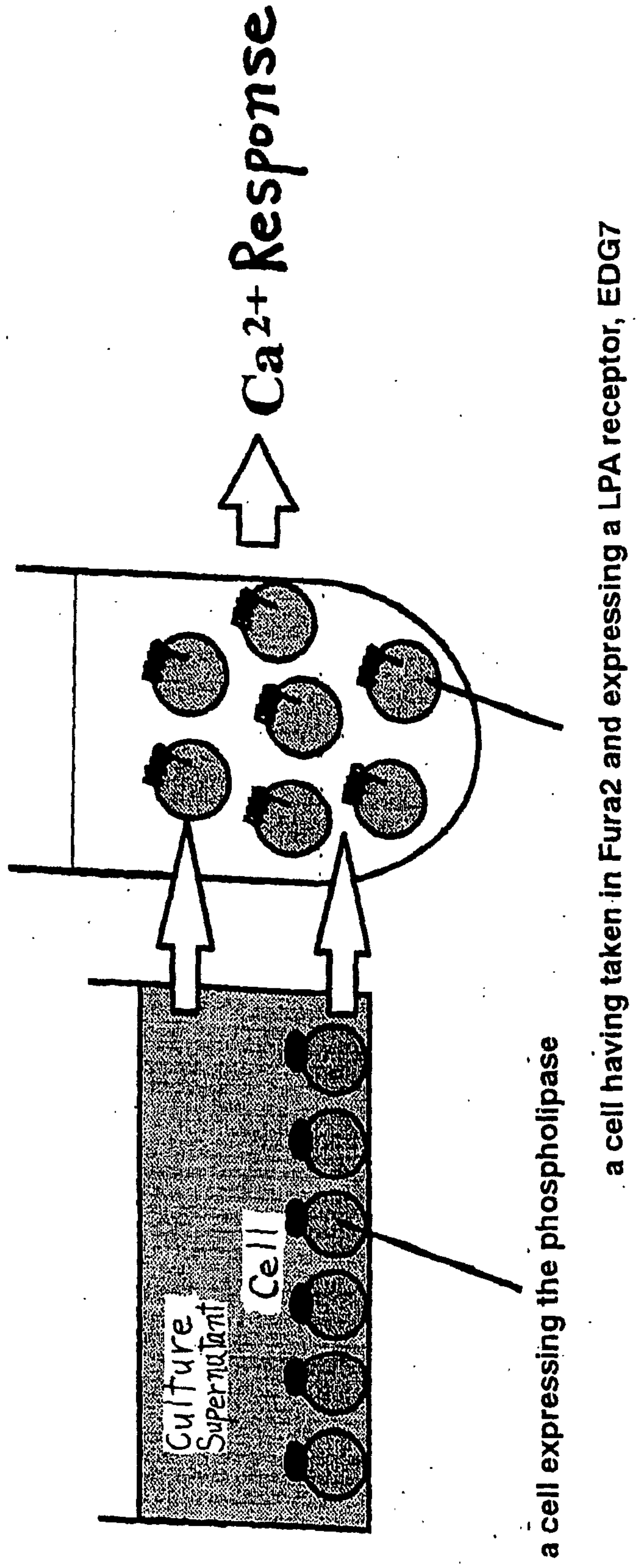

Ca2+ Response
Culture Supernatant
Cell
a cell expressing the phospholipase
a cell having taken in Fura2 and expressing a LPA receptor, EDG7


Fig. 3

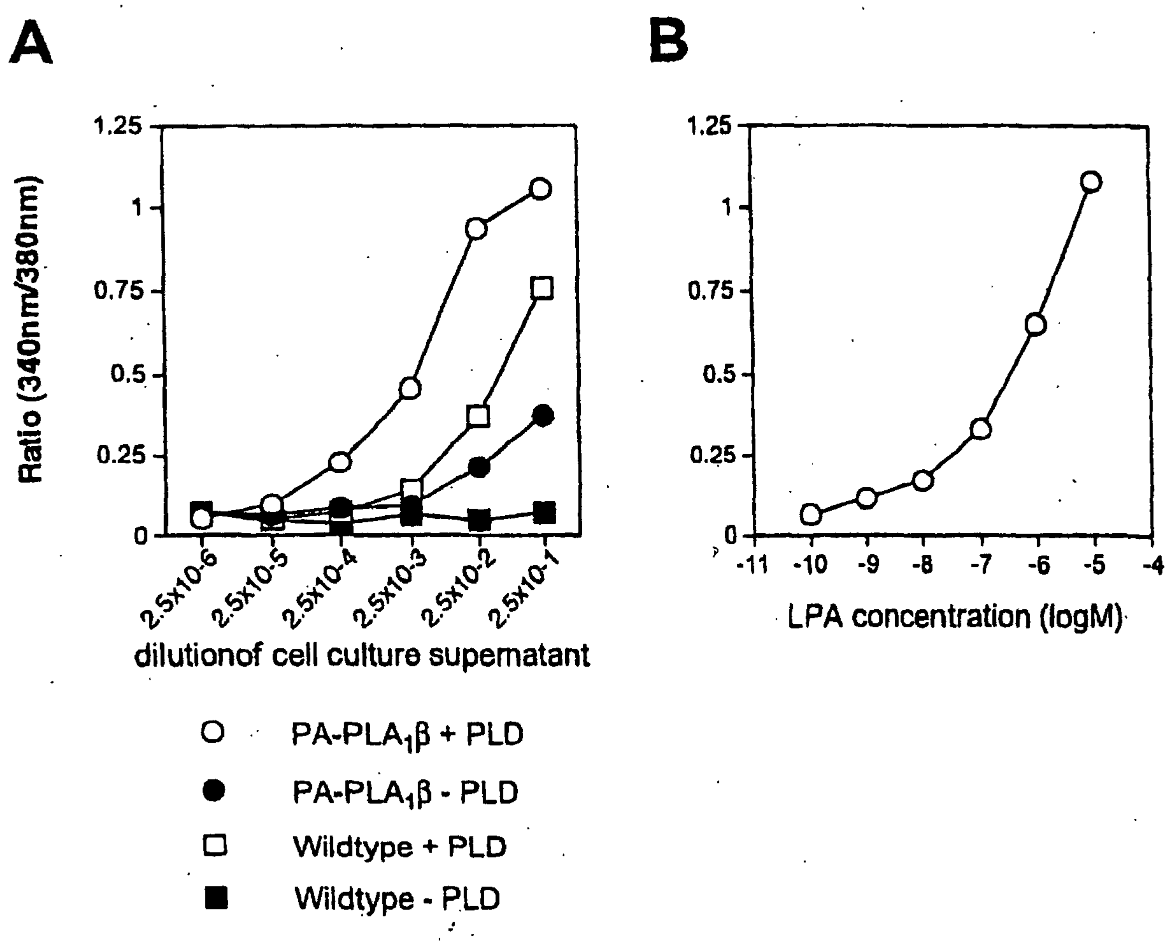
A
B
1.25
1
0.75
0.5
0.25
0
Ratio (340nm/380nm)
2.5x10-6
2.5x10-5
2.5x10-4
2.5x10-3
2.5x10-2
2.5x10-1
dilutionof cell culture supernatant
-11
-10
-9
-8
-7
-6
-5
-4
LPA concentration (logM)
PA-PLA1β + PLD
PA-PLA1β - PLD
Wildtype + PLD
Wildtype - PLD

Fig.4

## INTERNATIONAL SEARCH REPORT

International application No.
PCT/JP01/07106

A. CLASSIFICATION OF SUBJECT MATTER
Int.Cl[7] C12N 9/16, C12N 15/55, C12N 1/21, C12N 1/19, C12N 5/10, C07K 16/40, C12Q 1/02, C12Q 1/68, A61K 31/711, A61K 38/46, A61K 39/395, A61P 43/00

According to International Patent Classification (IPC) or to both national classification and IPC

B. FIELDS SEARCHED

Minimum documentation searched (classification system followed by classification symbols)
Int.Cl[7] C12N 9/16, C12N 15/55, C12N 1/21, C12N 1/19, C12N 5/10, C07K 16/40, C12Q 1/02, C12Q 1/68, A61K 31/711, A61K 38/46, A61K 39/395, A61P 43/00

Documentation searched other than minimum documentation to the extent that such documents are included in the fields searched

Electronic data base consulted during the international search (name of data base and, where practicable, search terms used)
GenBank/EMBL/DDBJ/GeneSeq, SwissProt/PIR/GeneSeq, MEDLINE (STN)

C. DOCUMENTS CONSIDERED TO BE RELEVANT

| Category* | Citation of document, with indication, where appropriate, of the relevant passages | Relevant to claim No. |
|---|---|---|
| X | WO 99/57132 A1 (GENETICS INST.INC.), 11 November, 1999 (11.11.99), & AU 9940711 A & EP 1077991 A1 | 1-9,11-12, 16,19 |
| X | JP 10-201479 A (Toray Industries, Inc.), 04 August, 1998 (04.08.98) (Family: none) | 2-9,11-12, 16,19 |
| X | WO 00/24911 A2 (INCYTE PHARM.INC.), 04 May, 2000 (04.05.00), & AU 200014516 A & EP 1131445 A1 | 2-9,11-12, 16,19 |
| P,X | WO 01/32885 A2 (MILLENNIUM PHARM.INC.), 10 May, 2001 (10.05.01), & AU 200113616 A | 2-9,11-12, 16,19 |
| A | SATO, T. et al., Serine phospholipid-specific phospholipase A that is secreted from activated platelets. A new member of the lipase family. J.Biol.Chem. 1997, Vol.272, No.4, pp.2192-8 | 1-13,16,19 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 November, 2001 (08.11.01) | 20 November, 2001 (20.11.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/07106

C (Continuation). DOCUMENTS CONSIDERED TO BE RELEVANT

| Category* | Citation of document, with indication, where appropriate, of the relevant passages | Relevant to claim No. |
|---|---|---|
| A | HIGGS, H. N. et al., Cloning of a phosphatidic acid-preferring phospholipase A1 from bovine testis. J.Biol.Chem. 1998, Vol.273, No.10, pp.5468-77 | 1-13,16,19 |

Form PCT/ISA/210 (continuation of second sheet) (July 1992)

## INTERNATIONAL SEARCH REPORT

International application No.
PCT/JP01/07106

**Box I Observations where certain claims were found unsearchable (Continuation of item 1 of first sheet)**

This international search report has not been established in respect of certain claims under Article 17(2)(a) for the following reasons:

1. ☒ Claims Nos.: 17,18
because they relate to subject matter not required to be searched by this Authority, namely:

Claim 17 pertains to diagnostic methods to be practiced on the human body, while claim 18 pertains to methods for treatment of the human body by therapy.

2. ☒ Claims Nos.: 14,15
because they relate to parts of the international application that do not comply with the prescribed requirements to such an extent that no meaningful international search can be carried out, specifically:

Although the compound as set forth in claim 14 involves in its scope any compounds identified by the methods as set forth in claims 11 to 13, no particular compound is presented in the description as the above-described compound. Thus, it is recognized the above claim is not sufficiently supported by the description. The same applies to claim 15.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ *No protest accompanied the payment of additional search fees.*

Form PCT/ISA/210 (continuation of first sheet (1)) (July 1992)

SEQUENCE LISTING

```
<110> MOCHIDA PHARMACEUTICAL CO., LTD.

<120> Novel PLA1

<130> N.88489 GCW

<140> EP 01956958.1
<141> 2001-08-20

<150> JP 2000-311015
<151> 2000-10-11

<160> 11

<210> 1
<211> 1677
<212> DNA
<213> Homo sapiens

<400> 1

ttttacagaa gaacctgcca gcctgtgatg atcctaccaa agagaaacct caatgagtta    60
tggaatttcc tttttggtga attgagtgct gtttttgctt ttctcagatt ccaa atg     117
                                                            Met
                                                            1

aga gta tac att ttt ctt tgt ttg atg tgc tgg gtg aga tct gat aat     165
Arg Val Tyr Ile Phe Leu Cys Leu Met Cys Trp Val Arg Ser Asp Asn
                5                   10                  15

aaa aga cca tgc ctt gaa ttc tct cag cta agt gta aag gat tcc ttc     213
Lys Arg Pro Cys Leu Glu Phe Ser Gln Leu Ser Val Lys Asp Ser Phe
        20                  25                  30

aga gat tta ttt att ccg aga ata gag acc att ctg atg atg tat aca     261
Arg Asp Leu Phe Ile Pro Arg Ile Glu Thr Ile Leu Met Met Tyr Thr
    35                  40                  45

agg aac aac cta aac tgt gct gag cca ctg ttt gaa caa aat aac tca     309
Arg Asn Asn Leu Asn Cys Ala Glu Pro Leu Phe Glu Gln Asn Asn Ser
50                  55                  60                  65

ctt aat gtt aat ttc aac aca caa aag aaa aca gtc tgg ctt att cac     357
Leu Asn Val Asn Phe Asn Thr Gln Lys Lys Thr Val Trp Leu Ile His
                70                  75                  80

gga tac aga cca gta ggc tcc atc cca tta tgg ctt cag aac ttc gta     405
Gly Tyr Arg Pro Val Gly Ser Ile Pro Leu Trp Leu Gln Asn Phe Val
            85                  90                  95

agg att ttg ctg aat gaa gaa gat atg aat gta att gta gta gac tgg     453
Arg Ile Leu Leu Asn Glu Glu Asp Met Asn Val Ile Val Val Asp Trp
        100                 105                 110

agc cgg ggt gct aca act ttt att tat aat aga gca gtt aaa aac acc     501
Ser Arg Gly Ala Thr Thr Phe Ile Tyr Asn Arg Ala Val Lys Asn Thr
    115                 120                 125
```

```
aga aaa gtt gct gtg agt ttg agt gtg cac att aaa aat ctt ttg aag     549
Arg Lys Val Ala Val Ser Leu Ser Val His Ile Lys Asn Leu Leu Lys
130                 135                 140                 145

cat ggt gca tct ctt gac aat ttt cat ttc ata ggt gtg agt tta ggg     597
His Gly Ala Ser Leu Asp Asn Phe His Phe Ile Gly Val Ser Leu Gly
                150                 155                 160

gct cat atc agt gga ttt gtt gga aag ata ttt cat ggt caa ctt gga     645
Ala His Ile Ser Gly Phe Val Gly Lys Ile Phe His Gly Gln Leu Gly
            165                 170                 175

aga ata aca ggt ctt gac cct gct ggg cca agg ttc tcc aga aaa cca     693
Arg Ile Thr Gly Leu Asp Pro Ala Gly Pro Arg Phe Ser Arg Lys Pro
        180                 185                 190

cca tat agc aga tta gat tac acg gat gca aag ttt gtg gat gtc atc     741
Pro Tyr Ser Arg Leu Asp Tyr Thr Asp Ala Lys Phe Val Asp Val Ile
    195                 200                 205

cat tct gac tcc aat ggt tta ggc att caa gag ccc ttg gga cat ata     789
His Ser Asp Ser Asn Gly Leu Gly Ile Gln Glu Pro Leu Gly His Ile
210                 215                 220                 225

gat ttt tat cca aat gga gga aat aaa caa cct ggc tgt cct aaa tca     837
Asp Phe Tyr Pro Asn Gly Gly Asn Lys Gln Pro Gly Cys Pro Lys Ser
                230                 235                 240

att ttc tca gga att caa ttc att aaa tgc aac cac cag aga gca gtt     885
Ile Phe Ser Gly Ile Gln Phe Ile Lys Cys Asn His Gln Arg Ala Val
            245                 250                 255

cac ttg ttc atg gca tct tta gaa aca aac tgc aat ttt att tca ttt     933
His Leu Phe Met Ala Ser Leu Glu Thr Asn Cys Asn Phe Ile Ser Phe
        260                 265                 270

cct tgt cgt tca tac aaa gat tac aag act agc tta tgt gtg gac tgt     981
Pro Cys Arg Ser Tyr Lys Asp Tyr Lys Thr Ser Leu Cys Val Asp Cys
    275                 280                 285

gac tgt ttt aag gaa aaa tca tgt cct cgg ctg ggt tat caa gcc aag    1029
Asp Cys Phe Lys Glu Lys Ser Cys Pro Arg Leu Gly Tyr Gln Ala Lys
290                 295                 300                 305

cta ttt aaa ggt gtt tta aaa gaa agg atg gaa gga aga cct ctt agg    1077
Leu Phe Lys Gly Val Leu Lys Glu Arg Met Glu Gly Arg Pro Leu Arg
                310                 315                 320

acc act gtg ttt ttg gat aca agt ggt aca tat cca ttc tgt acc tat    1125
Thr Thr Val Phe Leu Asp Thr Ser Gly Thr Tyr Pro Phe Cys Thr Tyr
            325                 330                 335

tat ttt gtt ctc agt ata att gtt cca gat aaa act atg atg gat ggc    1173
Tyr Phe Val Leu Ser Ile Ile Val Pro Asp Lys Thr Met Met Asp Gly
        340                 345                 350

tcg ttt tca ttt aaa tta tta aat cag ctt gga atg att gaa gag cca    1221
Ser Phe Ser Phe Lys Leu Leu Asn Gln Leu Gly Met Ile Glu Glu Pro
    355                 360                 365

agg ctt tat gaa aag aac aaa cca ttt tat aaa ctt caa gaa gtc aag    1269
```

```
Arg Leu Tyr Glu Lys Asn Lys Pro Phe Tyr Lys Leu Gln Glu Val Lys
370                 375                 380                 385

att ctt gct caa ttt tat aat gac ttt gta aat att tca agc att ggt   1317
Ile Leu Ala Gln Phe Tyr Asn Asp Phe Val Asn Ile Ser Ser Ile Gly
                390                 395                 400

ttg aca tat ttc cag agc tca aat ctg cag tgt tcc aca tgc aca tac   1365
Leu Thr Tyr Phe Gln Ser Ser Asn Leu Gln Cys Ser Thr Cys Thr Tyr
            405                 410                 415

aag atc cag aga ctc atg tta aaa tca ctt aca tac cca gaa aga cca   1413
Lys Ile Gln Arg Leu Met Leu Lys Ser Leu Thr Tyr Pro Glu Arg Pro
        420                 425                 430

cca ctt tgc agg tat aat att gta ctt aaa gac aga gag gaa gtg ttt   1461
Pro Leu Cys Arg Tyr Asn Ile Val Leu Lys Asp Arg Glu Glu Val Phe
    435                 440                 445

ctt aat cca aac aca tgt aca cca aag aac aca taa gatgccttct tccatc   1513
Leu Asn Pro Asn Thr Cys Thr Pro Lys Asn Thr
450                 455                 460

aaatgcactt gcttgtgaat taatggactt gtaaatgaaa caatgcaatc agtcttttat   1573
aatgcactgt tcaatttgag attcaagtat ttctatttct tggaaaaaat tttaagaatc   1633
aaaaataaag aaaataaaaa atgcatacag ttaaacattc caaa                    1677


<210> 2
<211> 1636
<212> DNA
<213> Homo sapiens

<400> 2

ggtcttattt atg ttg ctc aaa tgt tta cat aat aac ttg tgc caa aaa      49
           Met Leu Leu Lys Cys Leu His Asn Asn Leu Cys Gln Lys
             1               5                  10

tat agt gct cat gct ttt cag ttc tca ccc aga aat gtc ctg tgg ctt      97
Tyr Ser Ala His Ala Phe Gln Phe Ser Pro Arg Asn Val Leu Trp Leu
     15                  20                  25

cta gtt gtg tgc ctg aga tca gat aat aaa aga cca tgc ctt gaa ttc     145
Leu Val Val Cys Leu Arg Ser Asp Asn Lys Arg Pro Cys Leu Glu Phe
 30                  35                  40                  45

tct cag cta agt gta aag gat tcc ttc aga gat tta ttt att ccg aga     193
Ser Gln Leu Ser Val Lys Asp Ser Phe Arg Asp Leu Phe Ile Pro Arg
                50                  55                  60

ata gag acc att ctg atg atg tat aca agg aac aac cta aac tgt gct     241
Ile Glu Thr Ile Leu Met Met Tyr Thr Arg Asn Asn Leu Asn Cys Ala
            65                  70                  75

gag cca ctg ttt gaa caa aat aac tca ctt aat gtt aat ttc aac aca     289
Glu Pro Leu Phe Glu Gln Asn Asn Ser Leu Asn Val Asn Phe Asn Thr
        80                  85                  90

caa aag aaa aca gtc tgg ctt att cac gga tac aga cca gta ggc tcc     337
Gln Lys Lys Thr Val Trp Leu Ile His Gly Tyr Arg Pro Val Gly Ser
```

```
    95                  100                  105

atc cca tta tgg ctt cag aac ttc gta agg att ttg ctg aat gaa gaa       385
Ile Pro Leu Trp Leu Gln Asn Phe Val Arg Ile Leu Leu Asn Glu Glu
110                 115                  120                 125

gat atg aat gta att gta gta gac tgg agc cgg ggt gct aca act ttt       433
Asp Met Asn Val Ile Val Val Asp Trp Ser Arg Gly Ala Thr Thr Phe
                130                  135                 140

att tat aat aga gca gtt aaa aac acc aga aaa gtt gct gtg agt ttg       481
Ile Tyr Asn Arg Ala Val Lys Asn Thr Arg Lys Val Ala Val Ser Leu
            145                 150                  155

agt gtg cac att aaa aat ctt ttg aag cat ggt gca tct ctt gac aat       529
Ser Val His Ile Lys Asn Leu Leu Lys His Gly Ala Ser Leu Asp Asn
        160                 165                  170

ttt cat ttc ata ggt gtg agt tta ggg gct cat atc agt gga ttt gtt       577
Phe His Phe Ile Gly Val Ser Leu Gly Ala His Ile Ser Gly Phe Val
    175                 180                  185

gga aag ata ttt cat ggt caa ctt gga aga ata aca ggt ctt gac cct       625
Gly Lys Ile Phe His Gly Gln Leu Gly Arg Ile Thr Gly Leu Asp Pro
190                 195                  200                 205

gct ggg cca agg ttc tcc aga aaa cca cca tat agc aga tta gat tac       673
Ala Gly Pro Arg Phe Ser Arg Lys Pro Pro Tyr Ser Arg Leu Asp Tyr
                210                  215                 220

acg gat gca aag ttt gtg gat gtc atc cat tct gac tcc aat ggt tta       721
Thr Asp Ala Lys Phe Val Asp Val Ile His Ser Asp Ser Asn Gly Leu
            225                 230                  235

ggc att caa gag ccc ttg gga cat ata gat ttt tat cca aat gga gga       769
Gly Ile Gln Glu Pro Leu Gly His Ile Asp Phe Tyr Pro Asn Gly Gly
        240                 245                  250

aat aaa caa cct ggc tgt cct aaa tca att ttc tca gga att caa ttc       817
Asn Lys Gln Pro Gly Cys Pro Lys Ser Ile Phe Ser Gly Ile Gln Phe
    255                 260                  265

att aaa tgc aac cac cag aga gca gtt cac ttg ttc atg gca tct tta       865
Ile Lys Cys Asn His Gln Arg Ala Val His Leu Phe Met Ala Ser Leu
270                 275                  280                 285

gaa aca aac tgc aat ttt att tca ttt cct tgt cgt tca tac aaa gat       913
Glu Thr Asn Cys Asn Phe Ile Ser Phe Pro Cys Arg Ser Tyr Lys Asp
                290                  295                 300

tac aag act agc tta tgt gtg gac tgt gac tgt ttt aag gaa aaa tca       961
Tyr Lys Thr Ser Leu Cys Val Asp Cys Asp Cys Phe Lys Glu Lys Ser
            305                 310                  315

tgt cct cgg ctg ggt tat caa gcc aag cta ttt aaa ggt gtt tta aaa      1009
Cys Pro Arg Leu Gly Tyr Gln Ala Lys Leu Phe Lys Gly Val Leu Lys
        320                 325                  330

gaa agg atg gaa gga aga cct ctt agg acc act gtg ttt ttg gat aca      1057
Glu Arg Met Glu Gly Arg Pro Leu Arg Thr Thr Val Phe Leu Asp Thr
    335                 340                  345
```

```
agt ggt aca tat cca ttc tgt acc tat tat ttt gtt ctc agt ata att   1105
Ser Gly Thr Tyr Pro Phe Cys Thr Tyr Tyr Phe Val Leu Ser Ile Ile
350                 355                 360                 365

gtt cca gat aaa act atg atg gat ggc tcg ttt tca ttt aaa tta tta   1153
Val Pro Asp Lys Thr Met Met Asp Gly Ser Phe Ser Phe Lys Leu Leu
                370                 375                 380

aat cag ctt gga atg att gaa gag cca agg ctt tat gaa aag aac aaa   1201
Asn Gln Leu Gly Met Ile Glu Glu Pro Arg Leu Tyr Glu Lys Asn Lys
            385                 390                 395

cca ttt tat aaa ctt caa gaa gtc aag att ctt gct caa ttt tat aat   1249
Pro Phe Tyr Lys Leu Gln Glu Val Lys Ile Leu Ala Gln Phe Tyr Asn
        400                 405                 410

gac ttt gta aat att tca agc att ggt ttg aca tat ttc cag agc tca   1297
Asp Phe Val Asn Ile Ser Ser Ile Gly Leu Thr Tyr Phe Gln Ser Ser
    415                 420                 425

aat ctg cag tgt tcc aca tgc aca tac aag atc cag aga ctc atg tta   1345
Asn Leu Gln Cys Ser Thr Cys Thr Tyr Lys Ile Gln Arg Leu Met Leu
430                 435                 440                 445

aaa tca ctt aca tac cca gaa aga cca cca ctt tgc agg tat aat att   1393
Lys Ser Leu Thr Tyr Pro Glu Arg Pro Pro Leu Cys Arg Tyr Asn Ile
                450                 455                 460

gta ctt aaa gac aga gag gaa gtg ttt ctt aat cca aac aca tgt aca   1441
Val Leu Lys Asp Arg Glu Glu Val Phe Leu Asn Pro Asn Thr Cys Thr
            465                 470                 475

cca aag aac aca taa gatgccttct tccatcaaat gcacttgctt gtgaattaat g   1497
Pro Lys Asn Thr
        480

gacttgtaaa tgaaacaatg caatcagtct tttataatgc actgttcaat ttgagattca   1557
agtatttcta tttcttggaa aaaattttaa gaatcaaaaa taaagaaaat aaaaaatgca   1617
tacagttaaa cattccaaa                                                1636


<210> 3
<211> 1677
<212> DNA
<213> Homo sapiens

<400> 3

ttttacagaa gaacctgcca gcctgtgatg atcctaccaa agagaaacct caatgagtta     60
tggaatttcc tttttggtga attgagtgct gtttttgctt ttctcagatt ccaaatgaga    120
gtatacattt ttctttgttt gatgtgctgg gtgagatctg ataataaaag accatgcctt    180
gaattctctc agctaagtgt aaaggattcc ttcagagatt tatttattcc gagaatagag    240
accattctga tgatgtatac aaggaacaac ctaaactgtg ctgagccact gtttgaacaa    300
aataactcac ttaatgttaa tttcaacaca caaaagaaaa cagtctggct tattcacgga    360
tacagaccag taggctccat cccattatgg cttcagaact tcgtaaggat tttgctgaat    420
gaagaagata tgaatgtaat tgtagtagac tggagccggg gtgctacaac ttttatttat    480
aatagagcag ttaaaaacac cagaaaagtt gctgtgagtt tgagtgtgca cattaaaaat    540
cttttgaagc atggtgcatc tcttgacaat tttcatttca taggtgtgag tttaggggct    600
catatcagtg gatttgttgg aaagatattt catggtcaac ttggaagaat aacaggtctt    660
gaccctgctg ggccaaggtt ctccagaaaa ccaccatata gcagattaga ttacacggat    720
```

```
gcaaagtttg tggatgtcat ccattctgac tccaatggtt taggcattca agagcccttg    780
ggacatatag atttttatcc aaatggagga aataaacaac ctggctgtcc taaatcaatt    840
ttctcaggaa ttcaattcat taaatgcaac caccagagag cagttcactt gttcatggca    900
tctttagaaa caaactgcaa ttttatttca tttccttgtc gttcatacaa agattacaag    960
actagcttat gtgtggactg tgactgtttt aaggaaaaat catgtcctcg gctgggttat   1020
caagccaagc tatttaaagg tgttttaaaa gaaaggatgg aaggaagacc tcttaggacc   1080
actgtgtttt tggatacaag tggtacatat ccattctgta cctattattt tgttctcagt   1140
ataattgttc cagataaaac tatgatggat ggctcgtttt catttaaatt attaaatcag   1200
cttggaatga ttgaagagcc aaggctttat gaaaagaaca aaccatttta taaacttcaa   1260
gaagtcaaga ttcttgctca attttataat gactttgtaa atatttcaag cattggtttg   1320
acatatttcc agagctcaaa tctgcagtgt tccacatgca catacaagat ccagagactc   1380
atgttaaaat cacttacata cccagaaaga ccaccacttt gcaggtataa tattgtactt   1440
aaagacagag aggaagtgtt tcttaatcca aacacatgta caccaaagaa cacataagat   1500
gccttcttcc atcaaatgca cttgcttgtg aattaatgga cttgtaaatg aaacaatgca   1560
atcagtcttt tataatgcac tgttcaattt gagattcaag tatttctatt tcttggaaaa   1620
aattttaaga atcaaaaata aagaaaataa aaaatgcata cagttaaaca ttccaaa      1677
```

<210> 4
<211> 1636
<212> DNA
<213> Homo sapiens

<400> 4

```
ggtcttattt atgttgctca aatgtttaca taataacttg tgccaaaaat atagtgctca     60
tgcttttcag ttctcaccca gaaatgtcct gtggcttcta gttgtgtgcc tgagatcaga    120
taataaaaga ccatgccttg aattctctca gctaagtgta aaggattcct tcagagattt    180
atttattccg agaatagaga ccattctgat gatgtataca aggaacaacc taaactgtgc    240
tgagccactg tttgaacaaa ataactcact taatgttaat ttcaacacac aaaagaaaac    300
agtctggctt attcacggat acagaccagt aggctccatc ccattatggc ttcagaactt    360
cgtaaggatt ttgctgaatg aagaagatat gaatgtaatt gtagtagact ggagccgggg    420
tgctacaact tttatttata atagagcagt taaaaacacc agaaaagttg ctgtgagttt    480
gagtgtgcac attaaaaatc ttttgaagca tggtgcatct cttgacaatt ttcatttcat    540
aggtgtgagt ttaggggctc atatcagtgg atttgttgga aagatatttc atggtcaact    600
tggaagaata acaggtcttg accctgctgg gccaaggttc tccagaaaac caccatatag    660
cagattagat tacacggatg caaagtttgt ggatgtcatc cattctgact ccaatggttt    720
aggcattcaa gagcccttgg gacatataga tttttatcca aatggaggaa ataaacaacc    780
tggctgtcct aaatcaattt tctcaggaat tcaattcatt aaatgcaacc accagagagc    840
agttcacttg ttcatggcat ctttagaaac aaactgcaat tttatttcat ttccttgtcg    900
ttcatacaaa gattacaaga ctagcttatg tgtggactgt gactgtttta aggaaaaatc    960
atgtcctcgg ctgggttatc aagccaagct atttaaaggt gttttaaaag aaaggatgga   1020
aggaagacct cttaggacca ctgtgttttt ggatacaagt ggtacatatc cattctgtac   1080
ctattatttt gttctcagta taattgttcc agataaaact atgatggatg gctcgttttc   1140
atttaaatta ttaaatcagc ttggaatgat tgaagagcca aggctttatg aaaagaacaa   1200
accattttat aaacttcaag aagtcaagat tcttgctcaa ttttataatg actttgtaaa   1260
tatttcaagc attggtttga catatttcca gagctcaaat ctgcagtgtt ccacatgcac   1320
atacaagatc cagagactca tgttaaaatc acttacatac ccagaaagac caccactttg   1380
caggtataat attgtactta aagacagaga ggaagtgttt cttaatccaa acacatgtac   1440
accaaagaac acataagatg ccttcttcca tcaaatgcac ttgcttgtga attaatggac   1500
ttgtaaatga aacaatgcaa tcagtctttt ataatgcact gttcaatttg agattcaagt   1560
atttctattt cttggaaaaa attttaagaa tcaaaaataa agaaaataaa aaatgcatac   1620
agttaaacat tccaaa                                                   1636
```

<210> 5
<211> 25
<212> DNA
<213> Artificial Sequences

<400> 5

attttgttca aacagtggct cagca 25

<210> 6
<211> 25
<212> DNA
<213> Artificial Sequences

<400> 6

ttcaaacagt ggctcagcac agttt 25

<210> 7
<211> 33
<212> DNA
<213> Artificial Sequences

<400> 7

cgcggatcca tgttgctcaa atgtttacat aat 33

<210> 8
<211> 33
<212> DNA
<213> Artificial Sequences

<400> 8

cgcggatcca tgagagtata catttttctt tgt 33

<210> 9
<211> 38
<212> DNA
<213> Artificial Sequences

<400> 9

aaatatgcgg ccgcttatgt gttctttggt gtacatgt 38

<210> 10
<211> 26
<212> DNA
<213> Artificial Sequences

<400> 10

aaaaacacca gaaaagttgc tgtgag 26

<210> 11
<211> 25
<212> DNA
<213> Artificial Sequences

<400> 11

gcttgataac ccagccgagg acatg 25